# EUROPEAN PATENT APPLICATION

(11) **EP 4 443 438 A1**
(43) Date of publication of application: **09.10.2024**
(21) Application number: 24168204.6
(22) Date of filing: 03.04.2024
(51) Int. Cl.: G16H 10/60

(54) **DIGITAL SYSTEM, A DIGITAL PLATFORM AND METHOD OF USE**

(30) Priority: 05.04.2023 GB 202305064
(71) Applicant: Nuumad Ltd, London W1W 5PF (GB)
(72) Inventor: Mora, Judit, London, W1W 5PF (GB); Batten, Nicholas Jay, London, W1W 5PF (GB)
(74) Representative: Tomkinson, Alexandra

(57) **Abstract**

A digital platform is provided including at least one graphical user interface (GUI) for display on display means in use and for allowing input of data relating to an individual in use. The digital platform is arranged to generate a digital data record including individual specific data based on the data input into the GUI in use. At least a first database is associated with and/or communicatively coupled to the digital platform for storing the digital data record. At least a second database is associated with and/or communicatively coupled to the digital platform for storing further digital data which comprises non-individual specific digital data. Processing means are provided with and/or associated with the digital platform. The processing means is arranged to undertake one or more data matching processes between data in the digital data record in the at least first database and the non-individual specific digital data in the at least second database, such that, on detection of one or more data matches based on one or more pre-determined data criteria between the digital data record and the non-individual specific digital data, a digital schedule and/or digital plan of action specific to the individual is automatically generated that is based on the one or more data matches of the one or more pre-determined data criteria in the at least first and second databases.

## Description

The present invention relates to a digital system, a digital platform and/or to a method of use thereof. More particularly, the present invention relates to a digital health system, a digital health platform and/or to a method of use thereof.

Although the digital system and/or platform described herein relates almost exclusively to a digital health system and/or digital health platform for generating a travel vaccination treatment plan and/or for generating a vaccination record relating to one or more individuals, it will be appreciated by persons skilled in the art that the digital system and/or digital platform of the present invention could be used for the consultation, recordal, storage and/or generation of any schedule or plan relating to an individual for use in any application. For example, the system and/or platform could be used for patient allergy management, implementing a weight loss program, one or more beauty appointments, vaccinations, hormone replacement therapy, Attention Deficit Hyperactivity Disorder (ADHD) treatment, test and treat services, private vaccination services and/or the like.

The generation of digital health records for patients in a digital health system using a graphical user interface is known and is widely used in hospitals, general medical practices (GPs), care homes, schools, pharmacies and/or the like. The graphical user interfaces typically allows a user to manually input an individual's data into the system for creation of a patient's health record for storage and/or later retrieval. Interpretation of any data provided in the patient's health record and any treatment plan that may need to be generated therefrom typically has to be undertaken manually by a trained medical practitioner. A separate appointment system may sit alongside the digital health patients records to allow patient appointments to be manually booked. As such, conventional digital health systems typically have limited functionality and rely on an experienced medical practitioner to interpret the data provided in a patient's health record and to manually set treatment plans. Prior art systems are therefore very dependent on skilled users, are labour intensive and provide a relatively expensive solution.

It is therefore an aim of the present invention to provide a digital system and/or a method of use thereof that overcomes the abovementioned problems.

It is a further aim of the present invention to provide a digital health system and/or a method of use thereof that overcomes the abovementioned problems.

It is a yet further aim of the present invention to provide a digital platform and/or a method of use thereof that overcomes the abovementioned problems.

It is a yet further aim of the present invention to provide a digital health platform and/or a method of use thereof that overcomes the abovementioned problems.

It is a yet further aim of the present invention to provide a digital vaccination platform, a digital vaccination system, a digital travel vaccination platform, a digital travel vaccination system and/or a method of using the same.

According to one aspect of the present invention there is provided a digital platform; said digital platform including:
- at least one graphical user interface (GUI) for display on a display means in use and for allowing input of data relating to an individual in use;
- the digital platform arranged to generate a digital data record including individual specific data based on the data into the GUI in use;
- at least a first database associated with and/or communicatively coupled to the digital platform for storing the digital data record;
- at least a second database associated with and/or communicatively coupled to the digital platform for storing further data which comprises non-individual specific digital data; and
- processing means provided with and/or associated with the digital platform;
   wherein the processing means are arranged to undertake one or more data matching processes between data in the digital data record in the at least first database and the non-individual specific digital data in the at least second database, such that, on detection of one or more data matches based on one or more pre-determined data criteria between the digital data record stored and the non-individual specific data, a digital schedule and/or digital plan of action specific to the individual is automatically generated that is based on the one or more data matches of the one or more pre-determined data criteria in the at least first and second databases.

Thus, the system of the present invention has the advantage that the individual's digital schedule and/or plan of action is generated automatically based on matched pre-determined data between the individual's digital data record in the at least first database and the non-individual specific data stored in the at least second database. As such, the user and/or individual of the digital platform or system does not necessarily have to be a highly trained and/or a qualified individual to be able to generate the schedule or plan of action for the individual. Generation of the schedule or plan of action can therefore be undertaken quickly, efficiently and in a cost effective manner. As a result, implementation of the individual's schedule or plan of action can also be carried out in a more timely and cost effective manner. The digital platform or system of the present invention also provides much wider functionality, thereby making it a useful tool for a much wider range of applications.

Preferably the digital data record, the non-individual specific data and/or the digital schedule and/or digital plan of action is in the form of, includes or consists of one or more digital data files; digital data packets, and/or the like.

Preferably the processing means includes micro-processing means, software, hardware, computer hardware, one or more electronic chips, one or more computer programs and/or the like.

Preferably the at least first and at least second databases are different, separate and/or distinct databases.

In one embodiment the at least second database includes, comprises or consists of non-individual specific data.

Preferably each of the at least first and/or the at least second databases include multiple databases.

In one embodiment, where the at least second database comprises two or more databases, the one or more data matching processes undertaken by the processing means requires a data match to be identified between the digital data record and each of or at least two of two or more databases in order to automatically generate the digital schedule and/or digital plan of action; and/or a data match is required between at least two of the pre-determined data criteria in the at least first and second databases in order to automatically generate the digital schedule and/or digital plan of action.

In one embodiment if no data match is found, the digital schedule and/or digital plan of action is not generated.

In one embodiment a user and/or individual has to make a selection in the GUI in order for the matching process to be initiated and/or for the digital schedule and/or digital plan of action to be automatically generated.

In one embodiment, as soon as a user and/or individual has completed a form provided on the GUI, the matching process is initiated and/or the digital schedule and/or digital plan of action is automatically generated.

Preferably at least one of the first and second databases are located on a different computer, server, computer network and/or the like from the other of the at least first and second databases.

Preferably one or more algorithms are provided with and/or associated with the digital platform and/or processing means and are used to generate the digital schedule, digital vaccination schedule and/or digital plan of action for the individual.

Preferably the one or more algorithms allow the data matching or cross-matching of data contained in the at least first and second databases.

Preferably at least one exact or substantially exact data match is required between data contained in the first and at least second databases in order for the digital schedule and/or digital plan of action to be generated.

Preferably at least two exact or substantially exact data matches are required.

Preferably the data matching process starts as soon as a user or individual complete a risk assessment form or a questionnaire via the digital platform.

In one embodiment the generation of the digital schedule and/or digital plan of action is undertaken fully automatically once at least one data match has been made. However, in one embodiment, the generation of the digital schedule and/or digital plan of action may be partially automatic in that one or more further selection processes may be required to be made by the user or individual during the generation of the schedule or plan of action.

In one embodiment, wherein once at least one data match has been made between the digital data in the at least first and second databases, a sub-set of digital data is generated and/or identified and this sub-set of digital data is included in or forms at least part of the specific schedule and/or plan of action, forms a new digital data record specific to the individual and/or is added to and/or associated with the individual's digital data record.

In one embodiment the one or more data matching processes do not use probability, machine learning (ML), neural network links (NWL), data automation engines to analyse large data sets and/or artificial intelligence (AI) methods.

In one embodiment once the sub-set of digital data is identified, at least one further data matching process takes place in respect of the first and at least second databases and/or a further database.

In one embodiment the processing means and/or one or more algorithms allows the data in the at least first and/or at least second databases and/or the matched data to be ranked and/or ordered. For example, matched data can be ranked in order to generate the digital schedule and/or digital plan of action by suitable means, such as for example, by risk factors, perceived risk weighting, importance, chronologically, relevance and/or the like. For example, the data in the databases could also be ranked to ensure a match needs to takes place with some data criteria in preference to other data criteria, or the non-individual data could be ranked according to importance or importance of source and/or the like.

Preferably only the highest ranked or highest ordered data, or a pre-determined ranking or order of data, is used to provide the digital schedule and/or digital plan of action.

In one embodiment once the individual's digital schedule and/or digital plan of action has been generated, an individual and/or user of the digital platform can select, accept, adjust, modify and/or reject one or more parts of the schedule and/or plan of action, such as for example to optionally create a further or updated individual's schedule or plan of action.

Preferably the generated digital schedule and/or digital plan of action for the individual includes or consists of any or any combination of a textual plan, one or more recommended products, one or more recommended vaccines, one or more recommended treatment plans, one or more dates, times, appointments, consultations, number of vaccine doses, types of vaccine immunisation available, real time immunisation status and/or the like.

In one embodiment the generated digital schedule and/or digital plan of action is an interactive schedule that needs one or more further actions to be completed, actioned or selected via the digital platform in order for the schedule or plan to be completed and/or for a completion certificate to be issued by the digital platform.

Preferably the generated digital schedule and/or digital plan of action for the individual includes or consists of a plurality of dates, times, appointments, consultations, number of vaccination doses, types of vaccine immunisation available, real time immunisation status and/or the like.

In one embodiment the digital schedule, digital plan of action, dates and times of appointments and/or consultations calculated via the digital platform can then be linked or automatically inserted into a digital calendar of the individual and/or user of the digital platform. For example, any appointment dates can be automatically inserted into a user's or individual's electronic calendar or reminder system and/or digital data record.

Preferably the scheduled events contained in the digital schedule and/or digital plan of action relate directly or indirectly to the matched digital data.

Preferably data matches of at least two more pre-determined data criteria are required between the data in the at least first and second databases in order for the individuals specific digital schedule and/or digital plan of action to be generated.

Preferably the digital schedule and/or digital plan of action of the individual is saved as part of the individual's digital data record and/or is digitally linked thereto.

In one embodiment at least part of the digital schedule and/or digital plan of action for the individual is saved and/or recoded as part of a user's database, data record, calendar and/or the like, the user being different to the individual. For example, the individual could be a patient and the user could be a medical practitioner.

In one embodiment search means or a search function are provided on and/or associated with the digital platform, at least first database and/or at least second database to allow a user and/or individual to search data contained therein or associated therewith.

In one embodiment navigational means or a navigation function are provided on and/or associated with the digital platform to allow a user and/or individual to navigate through different web pages, sections, databases and/or the like of the digital platform.

In one embodiment the navigational means or function forces the user and/or individual to navigate through the digital platform in a particular order and/or route.

In one embodiment the navigational means or function allows the user and/or individual to navigate through the digital platform via a user/individual selected arbitrary route and/or order.

For example, the navigational means or function guides a healthcare professional through a consultation process with an individual to allow individual's data to be collected, such as for example, the collection of general patient data, the patient's future travel plans data, the patient's vaccine history, the patient medical history data and/or the like. This data collection typically takes place prior to any data matching process and/or the digital schedule and/or plan of action being generated by the digital platform.

In one embodiment the data input into the digital platform can be via the individual or patient themselves and/or via a user or third party, such as for example by a medical practitioner, healthcare professional, pharmacist and/or the like.

Preferably the input of data can take place as part of a consultation, such as face to face consultation between a medical practitioner and a patient, via telephone consultation, a video consultation and/or the like.

In one embodiment the individual and/or user is required to complete a risk assessment form via the graphical user interface to form at least part of the input data for the individual. Preferably the risk assessment form includes a plurality of questions the individual has to answer to form the input data.

In one embodiment the graphical user interface provides one or more drop down menus; text boxes, selectable buttons, selectable links, options and/or the like to allow a user and/or individual of the system to make selections and/or input data via the same.

In one embodiment a data summary or data dashboard can be displayed as part of the graphical interface to provide an easy to visualise means for a user and/or individual to assess the status of their data input, digital schedule and/or digital plan of action calculation and/or generation.

In one embodiment the digital platform allows the risk of malaria to be established for a particular individual and for malaria medication to be dispensed if required.

In one embodiment the digital platform can be used to establish if certification of an individual or patient's medical history, vaccinations and/or conditions is required and, if so, optionally allowing this certification to be issued. For example, certificates may be required for yellow fever vaccination, meningitis vaccination and/or polio vaccination.

In one embodiment the digital platform provides a selectable option to allow an individual or patient to defer a vaccination, scheduled action in the digital action plan and/or the like if required.

In one embodiment the digital platform is a digital health platform.

Preferably the individual is a patient.

Preferably the input individual's data includes any or any combination of an individual's personal data, an individual's gender, an individual's contact details, an individual's health data, an individual's health status, an individual's medical background, an individual's medical history, one or more medications the individual is taking, one or more countries and/or geographical regions the individual intends to travel to within a certain time frame and/or in the future or immediate future, one or more activities the individual intends to undertake within a certain time frame and/or in the future or immediate future, one or more accommodation types the individual intends to stay in within a pre-determined time frame and/or in the future or immediate future, one or more modes of travel the individual intends to use within a pre-determined time frame and/or in the future or immediate future, vaccination history data relating to the individual and/or the like.

Preferably the non-individual specific data relates to, consists of or includes any or any combination of pre-verified data, one or more health conditions, diseases, vaccines, medical treatments, pharmaceutical products, therapeutic products, pre-programmed treatment or vaccination protocols, health risks and/or the like; country and/or geographical region specific health conditions, diseases, vaccine health risks and/or the like; health conditions, diseases, medical treatments, pharmaceutical products, therapeutic products, vaccines, health risks and/or the like associated with one or more activities, one or more modes of travel; one or more types of accommodations, national and/or regional guidelines, national and/or regional statistics and for each of the items mentioned above the associated risk factors and/or the like.

Preferably the one or more data criteria used for the data matching process includes any or any combination of a country and/or geographical region, a health condition, a disease, a medical treatment, a pharmaceutical product, a therapeutic product, a medication, a vaccine, a health risk, a mode of travel, a type of accommodation, an activity, gender, an individual's age, health status, one or more medications and/or the like.

Preferably the non-individual specific data in the at least second database can include data from a plurality of different databases or data sources. For example, the non-individual specific data or data sources can include data from the National Travel Health Network and Centre (NaTHNaC), Summary of Product Characteristics (SPC) data, national and/or regional guidelines, national and/or regional statistics, national vaccination guidelines, Green Book data including the latest information on vaccines and vaccination procedures for vaccine preventable infectious diseases in the UK, Centres for Disease Control and Prevention (CDC) data, World Health Organization (WHO) data, pre-programmed vaccine data, pre-programmed treatment plans, pre-programmed vaccine treatment plans, Travax Data by Public Health Scotland and/or the like.

Preferably the one or more algorithms combines the matched non-individual specific data from the at least second databases to provide the individual's specific digital schedule and/or digital plan of action.

Further preferably the combination uses an exact data matching algorithm.

In one embodiment the one or more algorithms uses the matched data from the first and at least second databases to provide a risk assessment or identify one or more perceived risks to form at least part of the individual's specific digital schedule and/or digital plan of action.

Preferably the digital platform and/or graphical user interface can be used by the individual or patient, by a user other than the individual or patient and/or by both the individual or patient and a user other than the individual or patient. For example, the individual or patient may input at least some of the data into the graphical user interface, such as for example by completion of a risk assessment and/or questionnaire, and/or a medical practitioner may input the individual's or patient's data following and/or during a consultation with the individual or patient.

In one embodiment the one or more algorithms used by the digital platform calculate patient specific risks for travel diseases based on the individual/patient's intended travel destination(s) and/or medical history.

In one embodiment the one or more algorithms used by the digital platform calculate immunisation status, patient vaccine availability, schedule availability and/or the like based on a patient's vaccination history, relevance for a patient's intended travel destination and/or medical history.

In one embodiment the one or more algorithms used by the digital platform calculate an individual's/ patient's vaccine digital schedule and/or digital plan of action based on a patient's vaccination history, relevance for a patient's intended travel destination, medical history, vaccine SPCs, national vaccination guidelines, travel health guidelines.

Preferably the individuals/patient's vaccine digital schedule and/or digital plan of action includes recommended vaccines, vaccine appointment dates, times, costs and/or the like.

Preferably the one or more vaccines identified by the digital platform as being relevant and/or recommended for the individual set out in the schedule and/or plan of action may include a plurality of dosages and the platform is arranged to automatically calculate and schedule future appointments for each of the multiple vaccine dosages.

In one embodiment the digital platform and/or one or more algorithms used by the digital platform allows vaccines to be ordered and /or offered to patients via the platform, such as for example based on patient specific risks, medical and/or vaccination history, intended travel details and/or the like.

In one embodiment the one or more algorithms used by the digital platform calculate vaccine availability for repeat consultations based on a patient's vaccination history, vaccinations for the intended travel destination, vaccination schedules determined and selected during a consultation and/or medical history.

In one embodiment the one or more algorithms used by the digital platform forecast available vaccine schedules depending on a patient's prior vaccination history.

In one embodiment the patient's digital data record includes any or any combination of consultation data, vaccination history, intended travel plans, medical history, risk assessment, vaccine selection, patient consent, travel risks, vaccine batch number records and/or the like.

In one embodiment the patient's digital data record includes single or multiple consultation data (past, current and/or future patient immunisation status), vaccination history, travel plans, medical history, vaccine selection, patient consent, vaccine schedule selection, travel risks, vaccine batch number records and/or the like.

In one embodiment the digital platform provides an individual risk assessment or travel health risk assessment. Further preferably the individual risk assessment or travel health risk assessment is based on patient input data and/or the one or more algorithm calculations.

In one embodiment the digital platform provides real time or live data oversight and/or review capabilities.

In one embodiment an user and/or individual of the digital platform can override any recommendations, schedules and/or plans of actions generated by the platform.

In one embodiment the digital platform can include one or more accessible summary data pages for data stored on and/or created via the at least second database. For example, a cheat sheet can be generated for each disease and/or vaccine to allow a medical practitioner or individual to access the same for information.

In one embodiment the digital platform includes vaccine decisions captured on the individuals' data record and/or on the platform itself. For example, a medical practitioner could add notes as to why they didn't offer a patient a vaccine based on a particular reason, such as patient's medical history.

According to an aspect of the present invention there is provided a digital system, said digital system comprising:
an electronic device;
a display screen provided with and/or associated with said electronic device;
a digital platform which is provided on, associated with and/or communicatively coupled to the at least one electronic device;
said digital platform including:
   - at least one graphical user interface (GUI) for display on the display screen in use and for allowing input of data relating to an individual in use;
   - the digital platform arranged to generate a digital data record including individual specific data based on the data input into the GUI in use;
   - at least a first database associated with and/or communicatively coupled to the digital platform for storing the digital data record;
   - at least a second database associated with and/or communicatively coupled to the digital platform for storing further digital data which comprises non-individual specific digital data; and
   - processing means provided with and/or associated with the digital platform;
wherein the processing means is arranged to undertake one or more data matching processes between data in the digital data record in the at least first database and the non-individual specific digital data in the at least second database, such that, on detection of one or more data matches based on one or more pre-determined data criteria between the digital data record and the non-individual specific digital data, a digital schedule and/or digital plan of action specific to the individual is automatically generated that is based on the one or more data matches of the one or more pre-determined data criteria in the at least first and second databases.
According to a further aspect of the present invention there is provided a digital health platform;
said digital health platform including at least one graphical user interface (GUI) for display on a display means in use and for allowing input of data relating to a patient in use;
the digital health platform arranged to generate a digital data record including patient specific data based on data input into the GUI in use;
at least part of said at least one GUI for submitting data relating to at least one future planned travel destination and/or vaccination history associated with the patient;
at least a first database associated with and/or communicatively coupled to the digital health platform for storing the input patient's digital data record including the patient's at least one future travel destination and/or vaccination history;
at least a second database associated with and/or communicatively coupled to the digital health platform for storing further digital data relating to at least one or more general health conditions, vaccines, and/or health risk factors associated with one or more travel destinations; and
processing means provided with and/or associated with the digital health platform;
   wherein the processing means are arranged to undertake one or more data matching processes between data in the digital data record in the at least first database and the further digital data in the at least second database, such that, on detection of one or more data matches relating at least to the at least one future travel destination in the patient's digital data record in the at least first database and the same travel destination in the at least second database, it calculates and/or generates a digital treatment plan or vaccination treatment plan for the patient for their at least one future travel destination.

Preferably the treatment plan generated is a digital treatment plan or digital treatment plan data record and is automatically associated with and/or saved with the individual's digital data record and/or stored in the at least first database. As such, an individual's treatment plan can be easily retrieved from the system as and when required.

Preferably the data relating to non-user specific information, such as for example general health conditions, health risk factors and/or different travel destinations, are stored as digital records, digital health condition records, digital health risk factor records and/or digital travel destination records.

Preferably reference to a digital record herein is reference to a physical data packet.

Preferably the treatment plan includes any or any combination of the identification of one or more vaccinations that the individual will be required and/or recommended they have before they travel to the future travel destination; the identification of one or more medications, pharmaceutical and/or therapeutic formulations the individual should take or is recommended to take with them or gain access to before, during and/or after travel to their at least one future travel destination; the identification of one or more items to take with them to the individual's future travel destination, the identification of risk recommendations for non-vaccine preventable risks at the individual's travel destination and/or the like.

Preferably the digital system is arranged such that when an individual's treatment plan is generated, it takes into account individual's input data stored in the first database that may require an individual's treatment plan to be altered or changed. For example, if the individual's input data includes data relating to vaccinations the individual has previously had, this may change the treatment required in the resulting individual's treatment plan that is generated.

In one embodiment timing means or a timing device are provided in and/or associated with the digital platform and/or digital system to allow times and/or dates to be calculated or automatically calculated.

Preferably the timing means or timing device is arranged to allow manual calculation and/or automatic calculation of one or more time points, appointments and/or the like at which one or more treatments that might have been recommended or required in the individual's generated treatment plan to take place. For example, the timing means may be used to calculate a time period at which, or over which one or more vaccinations may be given to an individual.

Preferably the one or more time points relate to times and/or dates in the future from the time when the treatment plan, schedule and/or plan of action was generated.

Preferably the timing means or timing device is arranged to take into account treatment dates already provided in the individual's input data in the at least first database and calculate and/or re-calculate the one or more time points that might be recommended or required in the individual's generated treatment plan.

In one embodiment the digital system includes scheduling means or vaccine scheduling means such that once one or more time points for treatment of an individual vaccine number and/or frequency of dosage are calculated, these times points are manually and/or automatically scheduled in calendar means associated with the system for that individual.

In one embodiment the system can include means for identifying when at least part of an individual's treatment plan or action plan has been completed. Optionally, once identification of completion has been confirmed, the system could generate any necessary certification for supply, for example, to the individual.

In one embodiment confirmation of completion of at least part of an individual's treatment or action plan can result in the generation of a modification and/or extension of the individual's treatment or action plan. For example, once confirmation has been received by the system that the user has had a first vaccine, a second vaccine and/or treatment course may then be suggested or scheduled by the system.

In one embodiment the digital platform, system and/or one or more databases can include cost information for one or more treatments, vaccines, medications recommended and/or required in the treatment plan or action plan.

In one embodiment payment information and/or processing means can be provided via the digital system or platform to allow payment advice or transactions for a recommended and/or required treatment set out in an individual's treatment plan or action plan to be taken.

In one embodiment the graphical user interface requires individual consent means that have to be selected and/or completed in order for or prior to an action plan, treatment recommended and/or required on the treatment plan to be carried out.

In one embodiment the graphical user interface can be accessed via any or any combination of a medical practitioner, health care practitioner, pharmacist, one or more individual's, vaccination provider and/or the like.

Preferably the individual's input data stored on the at least first database is in the form of a medical record or medical record data packet.

Preferably a plurality of individual's input data and/or medical records or medical record data packets are stored on the at least first database.

In one embodiment the display means is a display screen, a touch screen display and/or the like and data can be input into the graphical user interface via said screen display. In addition to and/or as an alternative, other user input means are provided to allow input of data into the graphical user interface.

Preferably the user input means can include any or any combination of a keyboard, a mouse, a joystick, a control unit, voice control means and/or the like.

In one embodiment the electronic device includes micro-processing means for processing and/or actuating one or more actions, steps, processes of the system and/or platform.

Preferably the electronic device and/or micro-processing means includes any or any combination of a computer, laptop, handheld electronic device, mobile phone, tablet device and/or the like.

In one embodiment the at least first and/or second databases can be stored in digital data storage means on a computer, memory, server, master-slave computer arrangement, in the cloud and/or the like.

In one embodiment the digital system includes search means or a search facility to allow data, one or more data records and/or the like to be identified and/or retrieved in use.

In one embodiment reference to communicatively coupled refers to communication via wired or wireless means, via the Internet, WIFI, Bluetooth, Near Field Communication (NFC), Infrared, radio waves and/or the like.

Preferably the data matching process takes place on one digital data record at any one time.

Preferably the generated digital schedule and/or digital plan of action is arranged to be displayed on a display screen in use.

In one embodiment the digital system and/or digital platform includes digital securing means, such as for example one or more encryption tools and/or the like to ensure data and/or data records can only be accessed via authorized individuals using identification means.

According to a further aspect of the present invention there is provided a method of using a digital platform, said digital platform including:
at least one graphical user interface (GUI) for display on display means in use and
for allowing input of data relating to an individual in use; at least a first database associated with and/or communicatively coupled to the digital platform; at least a second database associated with and/or communicatively coupled to the digital platform for storing further digital data which comprises non-individual specific digital data; processing means provided with and/or associated with the digital platform;
said method including the steps of:
   - inputting data relating to an individual into the GUI;
   - generating a digital data record including individual specific data based on the data input into the GUI;
   - storing the digital data record in the at least first database;
wherein the processing means carries out the method steps of:
   - undertaking one or more data matching processes between data in the digital data record in the at least first database and the non-individual specific digital data in the at least second database and, on detecting one or more data matches based on one or more pre-determined data criteria between the digital data record and the non-individual specific digital data, automatically generating a digital schedule and/or digital plan of action specific to the individual that is based on the one or more data matches of the one or more pre-determined data criteria in the at least first and second databases.

According to further aspects of the present invention there is provided a method of using a digital system, digital platform, digital health system or digital health platform.

It can therefore be seen that, in one embodiment, the present invention provides an electronic consultation and health record platform that can be used to determine travel specific risks, vaccine availability and vaccine schedules for an individual or patient. A healthcare professional can offer vaccine choices to a patient or individual based on data provided by, for example, the NaTHNaC, SPC data, Greenbook data, CDC data, WHO data and/or the like, and enables the health care professional to dispense vaccinations via a patient group direction (PGD) authorisation framework working on the basis of group exclusions. The digital health platform of the present invention can capture all the decisions made and creates an electronic health record for the patient or individual. The electronic health record can be used for repeat consultations with the patient or individual carried out via the digital health platform.

In one example, use of the digital platform excludes the creation of individual medical prescriptions.

In one embodiment the digital health record produced using the digital health platform for a patient or individual can be synchronised with one or more other digital health records for the individual or patient, such as for example with their National Health Service (NHS) immunisation records.

Embodiments of the present invention will now be described with reference to the following images, wherein:
Figure 1 is an example of a screenshot of a graphical user interface of a digital health platform for use by a medical practitioner showing the appointments scheduled in for the medical practitioner according to an embodiment of the present invention;
Figure 2 is an example of a screenshot of a graphical user interface of a digital health platform for use by a medical practitioner showing the addition of patient information onto the platform database according to an embodiment of the present invention;
Figure 3 is an example of a screenshot of a graphical user interface of a digital health platform for use by a medical practitioner showing how patient information is found that has already been added onto the platform database according to an embodiment of the present invention;
Figure 4 shows an example of a screenshot of a graphical user interface of a digital health platform for use by a medical practitioner showing a risk assessment summary for a selected patient according to an embodiment of the present invention;
Figure 5 is an example of a screenshot of a graphical user interface of a digital health platform for use by a medical practitioner showing examples of medical questions that can form the basis of a risk assessment used to form the summary in figures 4a and 4b according to an embodiment of the present invention;
Figure 6 is an example of a screenshot of a graphical user interface of a digital health platform for use by a medical practitioner used to record and/or display a patient's vaccine history according to an embodiment of the present invention;
Figure 7 is an example of a screenshot of a graphical user interface of a digital health platform for use by a medical practitioner showing an example of how travel plans for a patient can be input onto the platform database according to an embodiment of the present invention;
Figure 8 shows an example of a screenshot of a graphical user interface of a digital health platform for use by a medical practitioner showing an example of patient specific travel vaccination and travel risk advice that can be generated by the platform for a selected patient in respect of data input onto the platform for that patient according to an embodiment of the present invention;
Figure 9 is an example of a screenshot of a graphical user interface of a digital health platform for use by a medical practitioner showing how vaccines can be selected based on the travel advice generated by the platform as per figures 8a and 8b according to an embodiment of the present invention;
Figure 10 is an example of a screenshot of a graphical user interface of a digital health platform for use by a medical practitioner showing how confirmation of a vaccine being administered can be recorded on the platform in respect of a selected patient according to an embodiment of the present invention;
Figure 11 is an example of a screenshot of a graphical user interface of a digital health platform for use by a medical practitioner showing how a vaccination schedule can be set up for a selected patient on the platform according to an embodiment of the present invention;
Figure 12 is an example of a screenshot of a graphical user interface of a digital health platform for use by a medical practitioner showing a record vaccination for a selected patient on the platform according to an embodiment of the present invention;
Figure 13 is an example of a screenshot of a graphical user interface of a digital health platform for use by a medical practitioner showing how other risk advice, such as non-vaccine preventable risk advice, can be generated on the platform for a selected patient according to an embodiment of the present invention;
Figure 14 is an example of a screenshot of a graphical user interface showing the consultation data record generated by the digital health platform;
Figure 15 is an example of a screenshot of a graphical user interface showing the patient data record generated by the digital health platform;
Figure 16 is a schematic showing the data matching and decision determination process undertaken by the digital health platform in one embodiment;
Figure 17 is an example of a screenshot of a graphical user interface showing malaria risk assessment and medication recommendations in one embodiment.

The following figures show examples of screen shots that can form part of a graphical user interface for a digital health platform according to embodiments of the present invention. The digital health platform forms part of a digital health system which, in the following illustrated examples, is for providing a vaccination treatment plan based on future travel plans of an individual patient. The system uses one or more algorithms to automatically generate a vaccination treatment plan specific to the patient based on identification of a match between data contained on the patient's digital data record including the destination country or countries the patient intends to travel to in the near future and data that is stored as part of a patient's digital medical record, and digital data records of health conditions, vaccines and/or health risks associated with a plurality of different countries provided on a plurality of different databases or sources. A calendar and/or timing means is associated with the platform to allow dates and times for selected vaccines to be manually and/or automatically scheduled for the patient.

Although the user of the platform in the illustrated examples is described as a medical practitioner, it will be appreciated that any individual that is able to administer vaccinations could use the system.

Furthermore, although the following description refers almost exclusively to the generation of patient vaccination treatment plans, it will be appreciated that the digital platform could be used for any application as previously mentioned.

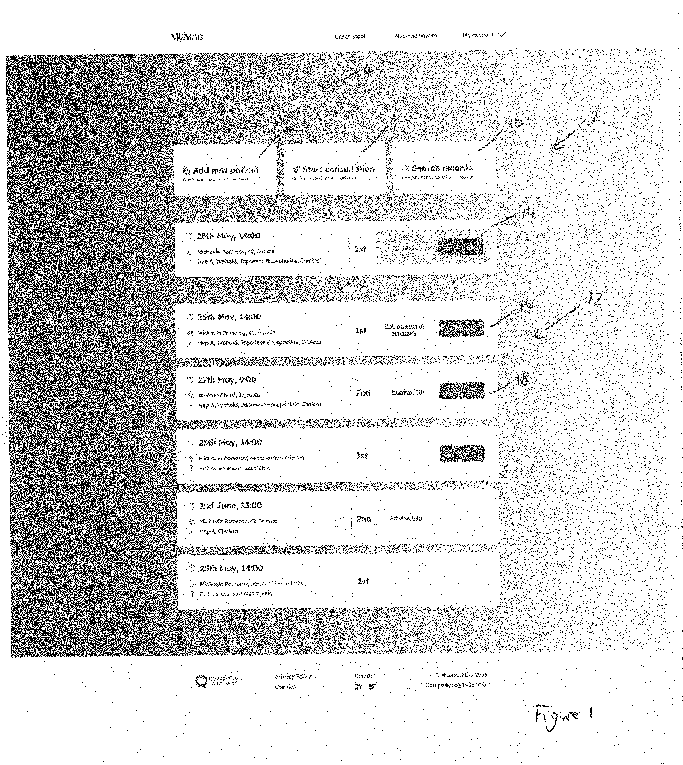

Referring to figure 1, there is illustrated an example of a screenshot of a graphical user interface 2 of a digital health platform. The name of the user or medical practitioner of the platform, which in this example case is "Laura", is presented in a welcome message 4 at the top of the page.

The interface 2 includes user selectable buttons for allowing adding a new patient 6, starting a new consultation 8, and searching existing digital patient records 10. An existing appointment schedule 12 for the user is provided on the page and lists the patients due to be seen by the user for a particular time and/or date range. More particularly, in this example, each appointment contained in the schedule for the particular time and/or date range is provided as a selectable button 14, 16, 18 with summary information provided as to the patient's name, age, gender, vaccines due, date and time of the appointment. The user can select any of buttons 14, 16, 18 to navigate to the patient's data record.

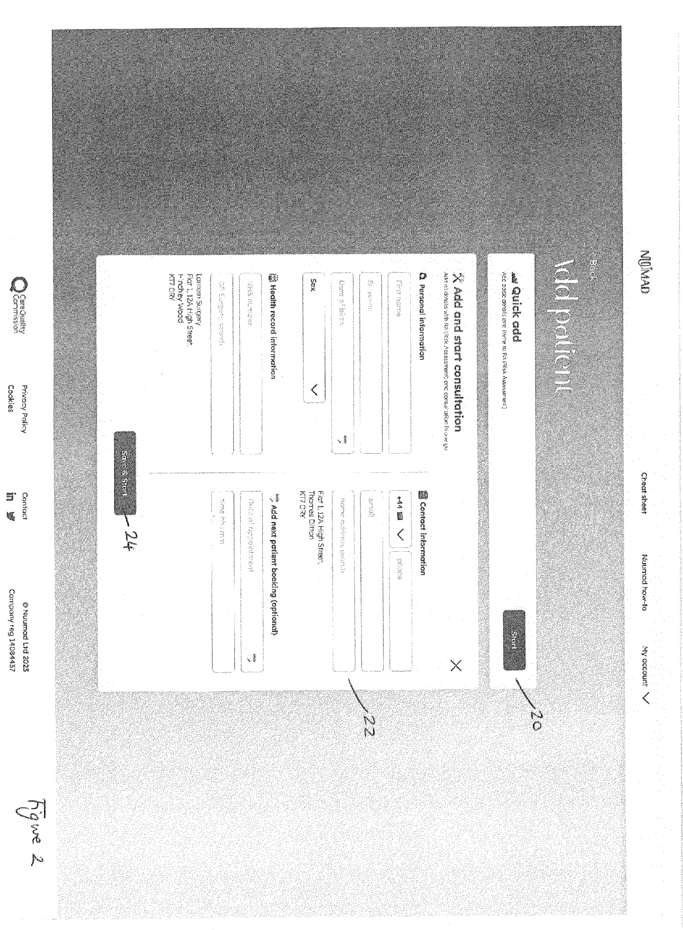

Referring to figure 2, there is illustrated a further example of a screenshot of the graphical user interface 2 by which the user can add a patient to the digital platform and create a digital data record for storage in a first or patient record database associated with the platform. There is a user selectable button 20 to allow the quick addition of a patient onto the system to form a digital patient record, and a form 22 is provided that can be populated by a user to generate a more detailed patient digital record. Selection of this buttons also sends a risk assessment questionnaire to the patient for completion by the patient, thereby saving time in the consultation with the health care professional. For example, the risk assessment questionnaire sent to the patient could include patient data, patient contact data, patient GP data, travel destination data, vaccine history data, medical history data and/or the like. The form 22 is typically completed by the user as part of a "consultation" with a patient. In this example, the form includes the user's personal information, such as name, surname, date of birth, gender, contact details, current GP details and an option to make a manually generated appointment for the patient if required using the calendar of the system. A button 24 is provided to save the input patient data and therefore generate a digital patient record, typically comprising a digital data packet, for that patient which is stored on the patient record database.

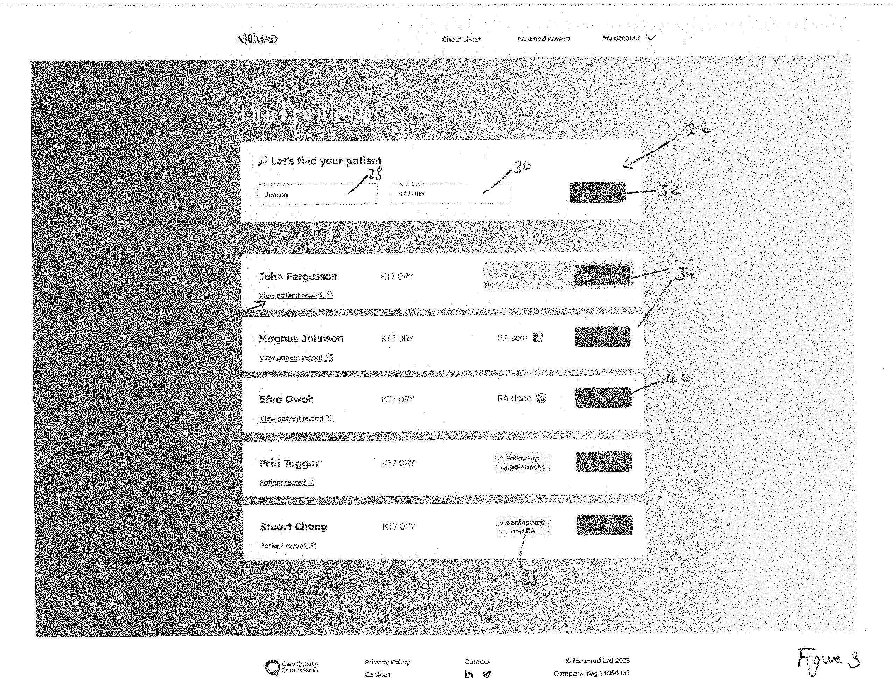

Figure 3 is an example of a screenshot of the graphical user interface 2 by which a user can find a digital patient record already contained within the patient record database. There are form boxes 26 for finding a patient by name 28 and post code 30 and a user selectable search button 32. Any potentially relevant patient results identified from the search can be displayed on the page as separate selectable patient records 34. The records 34 typically provide a brief summary of the patient identified, together with an indication as to whether the patient has completed a risk assessment (RA) form as part of their record. A link 36 can be provided to view the detailed patient record and further selectable buttons 38, 40 can be provided to send and/or initiate a risk assessment or to start a detailed consultation respectively.

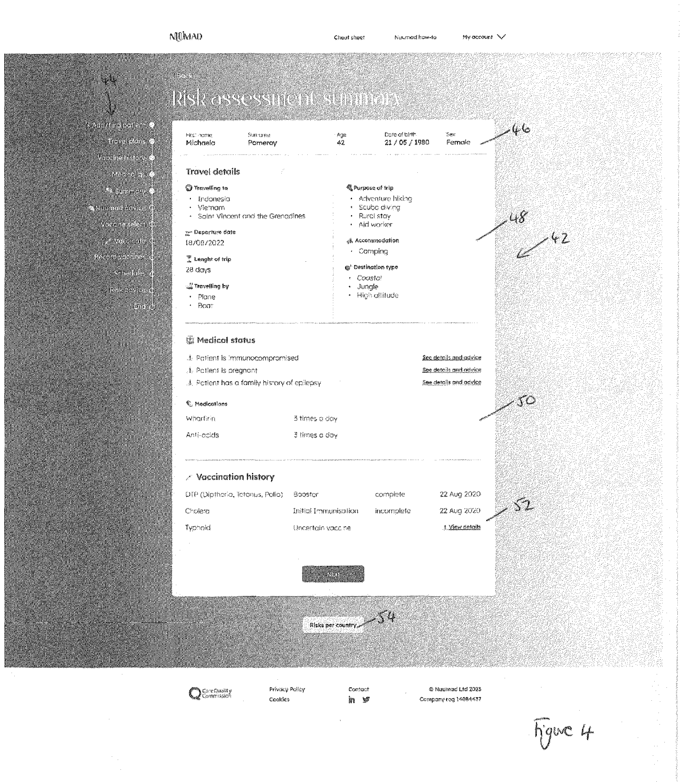

A risk assessment (RA), in one embodiment of the present invention, typically needs to be completed by the user via the platform as part of the consultation to form part of the digital patient's record. An example of a risk assessment summary 42 for a particular patient "Michaela Pomeroy" shown as a screenshot of the graphical user interface 2 is illustrated in figure 4.

A summary menu or navigational dashboard 44 is provided to allow rapid visualisation by a user of the stages that have been completed in the vaccination process. Different icons can be displayed (or a button filled or left empty in this example) to show whether a task has been performed or not. For example, the summary menu can include details of tasks including whether the patient has been added, whether patient travel destination data has been input, whether patient vaccine history has been input, whether a patient medical questionnaire has been completed, the provision of a patient risk assessment summary, the provision of patient travel advice, whether vaccines have been selected, the vaccination itself, vaccine record, schedules for future appointments, records of vaccines given, further risk advice on the travel destination and/or the like. The dashboard can allow a user to move backwards and forwards through the navigational process quickly and efficiently as required.

The summary 42 includes a section 46 containing the name, age, date of birth and gender of the patient; a section 48 containing details of the patient's imminent travel plans, such as for example, the travel destination, the departure date, the length of the trip, the mode of transport, the purpose of the trip, the type of accommodation to be used during the trip, the type of travel destination and/or the like; a section 50 on the patient's medical status, such as for example any medical conditions that need to be considered when providing vaccination advice, any current medications the patient is on and/or the like; a section 52 is provided setting out the vaccination history of the patient.

A user selectable button 54 can be provided to identify the risks per country provided on the risk summary list of countries the patient intends to travel to.

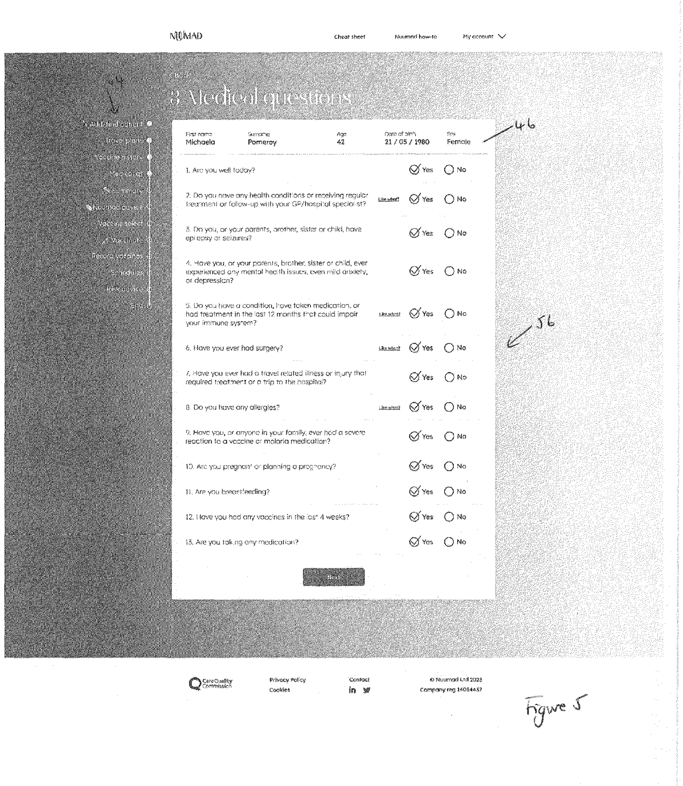

Figure 5 shows an example of a medical history form 56 that can be used as part of the risk assessment for a patient for completion by a user of the platform. The form can contain a simple tick box list of questions with answers of either "yes" or "no" to keep the form as simple as possible in one example.

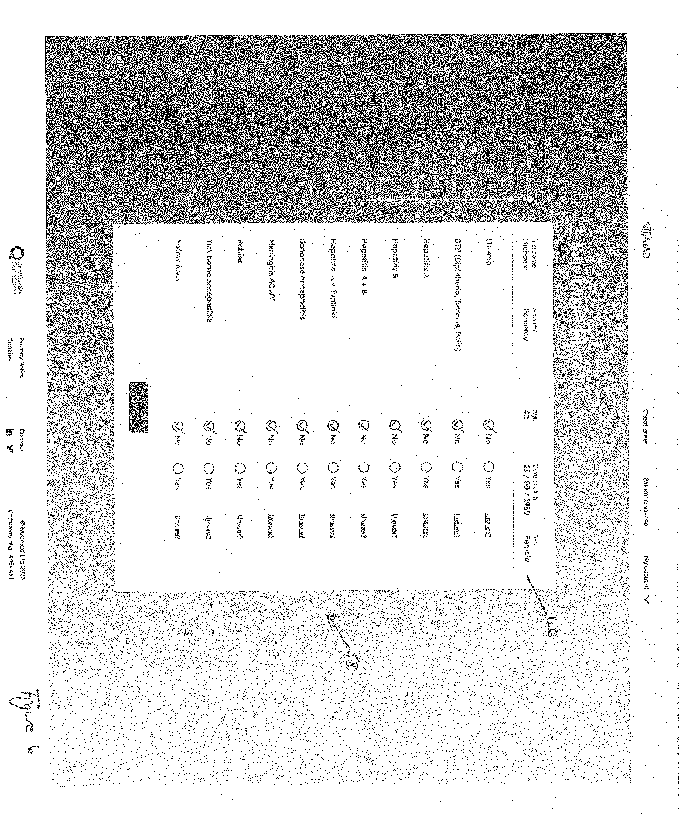

Figure 6 shows an example of a patient's vaccine history screenshot 58 on the graphical user interface 2. A list of possible vaccines can be provided and tick boxes of either "yes" or "no" could be provided next to each vaccine to provide an easy visualisation summary of the patient's vaccine history.

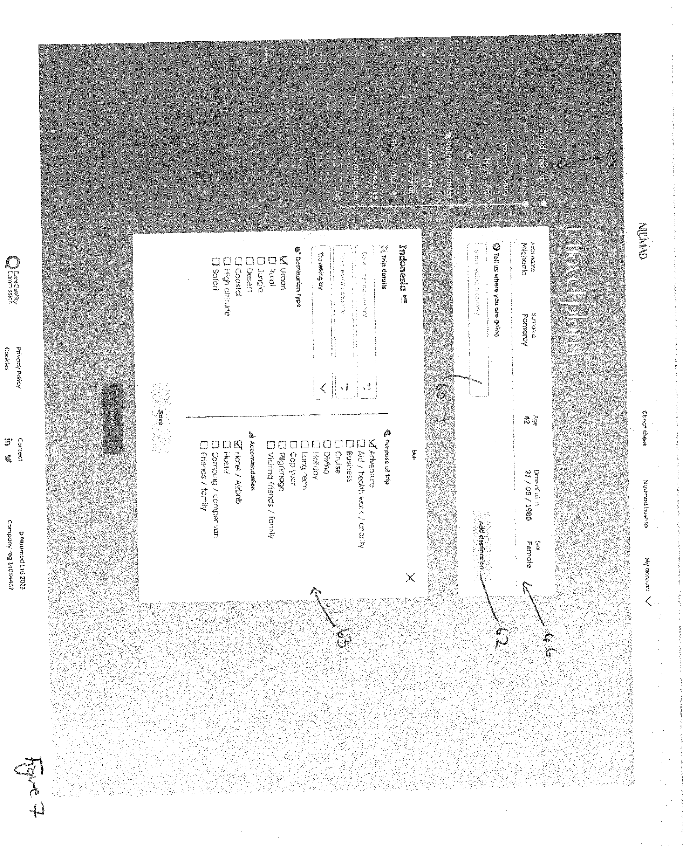

Figure 7 shows an example of a screenshot on the graphical user interface 2 for searching for the countries the patient intends to travel to in the future or imminent future. A search box 60 is provided to allow a user to type in the country in the country the patient intends to travel to and a user selectable button 62 is provided to allow the country to be added to the patient's digital record. A section 63 with user selectable options for the chosen country is shown so that the purpose of the trip can be identified, the terrain within the country that is to be visited by the patient and the accommodation that the patient will stay in during their trip.

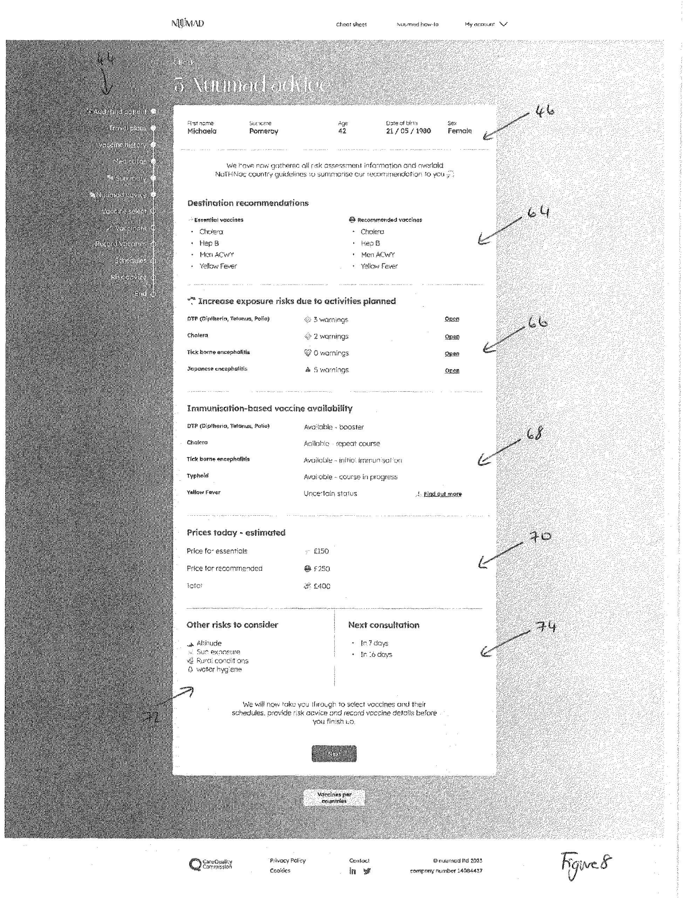

Figure 8 shows an example screenshot on the graphical user interface 2 for the treatment plan or vaccination advice that can be automatically generated by the digital health platform as a result of a match being identified between the country or countries on the digital patient medical record that has been stored on the system in a first database and the health risks and conditions associated with that country stored on at least a second database. One or more algorithms perform the matching or cross matching process and to generate the resulting recommendations, treatment plan or action plan for the patient.

A section 64 identifies the vaccines that are essential for travel to the particular travel destination and the vaccines that are recommended for travel to the patient's travel destination; a section 66 is provided to identify the increased risk of exposure of the patient to certain health conditions or diseases in the travel destination country based on the activities that the patient indicated they were intending to undertake during their travels; a section 68 is provided to set out the immunisation based vaccine availability for the vaccines identified as being recommended or essential; a section 70 is provided to set out the costs for the vaccinations required; a section 72 sets out other risk factors the patient may need to take into consideration when travelling to the travel destination, such as for example, altitude, sun exposure, rural conditions, water hygiene and/or the like; a schedule of appointments for the vaccines is automatically generated and shown in section 74.

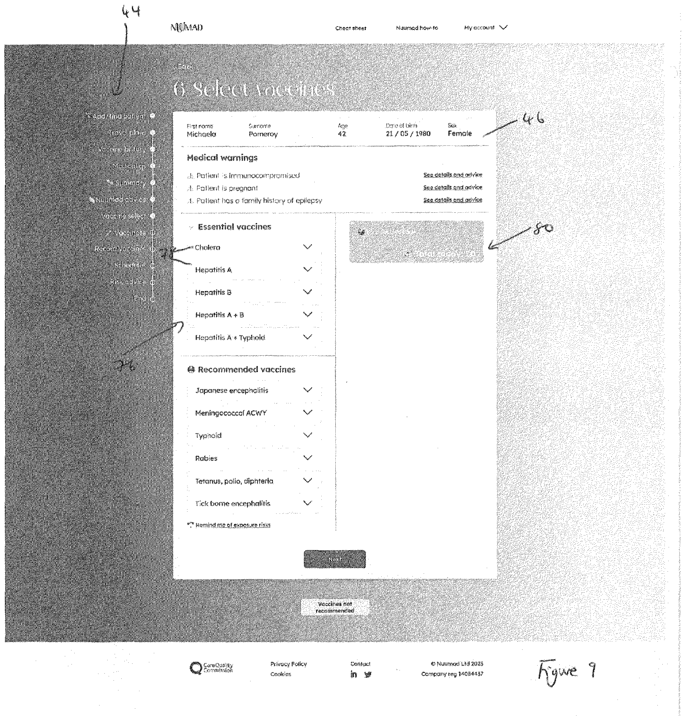

Figure 9 is an example of a screenshot on the graphical user interface 2 for allowing a user to select the vaccines for administering to the patient and that require payment by the patient. Thus, a section 76 of the user interface includes one or more user selectable boxes 78 relating to each vaccine that has been identified during the matching process as being essential for travel to the matched travel destination and recommended for travel to the matched travel destination. In order for a user to select the vaccine, they simply need to click on the relevant vaccine box 78. Once a suitable vaccine box has been selected, a cost calculation selection 80 is automatically completed and the vaccine is added to a basket for purchase and the cost of the selected vaccine is displayed. An option for the health care professional to add notes can be provided, such as for example if a patient decides not to proceed with a vaccine.

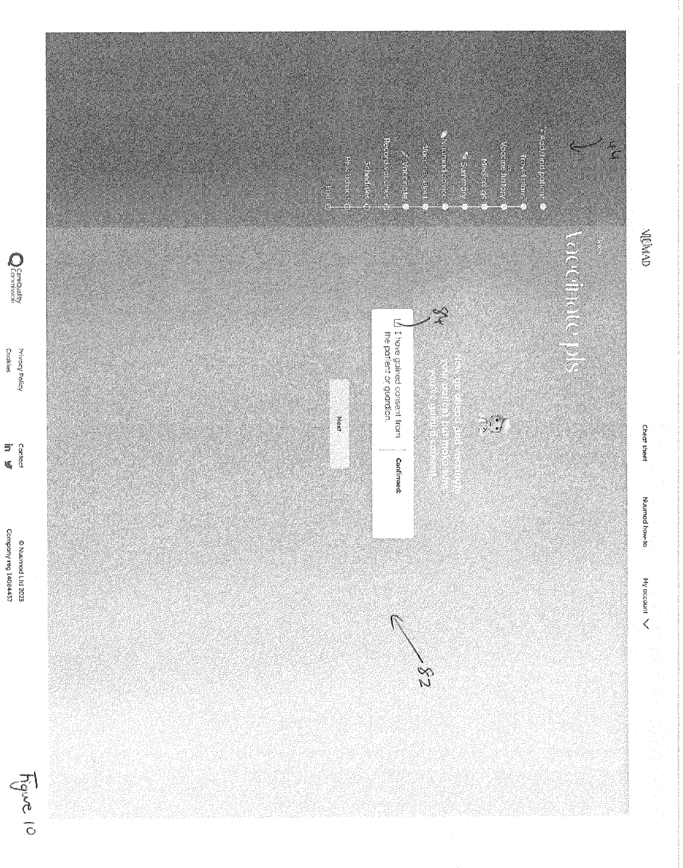

In the event that a patient decides to proceed with a selected vaccination, the user needs to obtain consent from the patient and an example of a screenshot taken from a graphical user interface 2 showing a consent page 82 is shown in figure 10. The user has to positively select a consent tick box 84 to confirm they have obtained consent from the patient.

It will be appreciated that the user and/or patient can adjust, change, delete and/or add to the recommended treatment plan generated by the system.

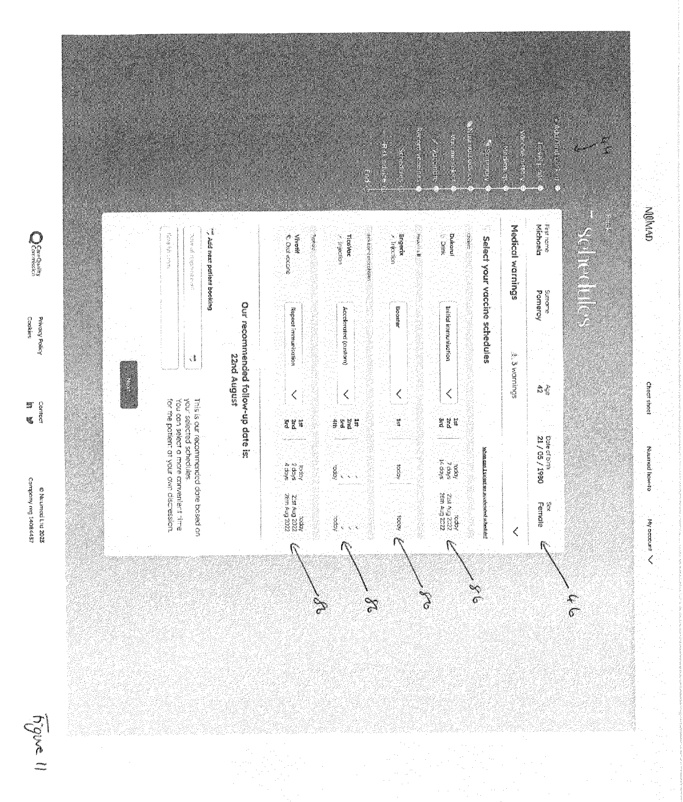

Figure 11 is an example of a screenshot on the graphical user interface 2 showing a vaccine schedule that is automatically created by the digital health platform in response to the vaccine selection made on the vaccine selection page shown in figure 9. The user interface provides a number of vaccine boxes; one for each selected vaccine 86. The vaccine box indicates the vaccine in question, the status, the number of dosages of the vaccine required to be administered and/or stage of vaccination and the calculated dates for when the vaccine and/or any subsequent vaccine boosts need to be administered.

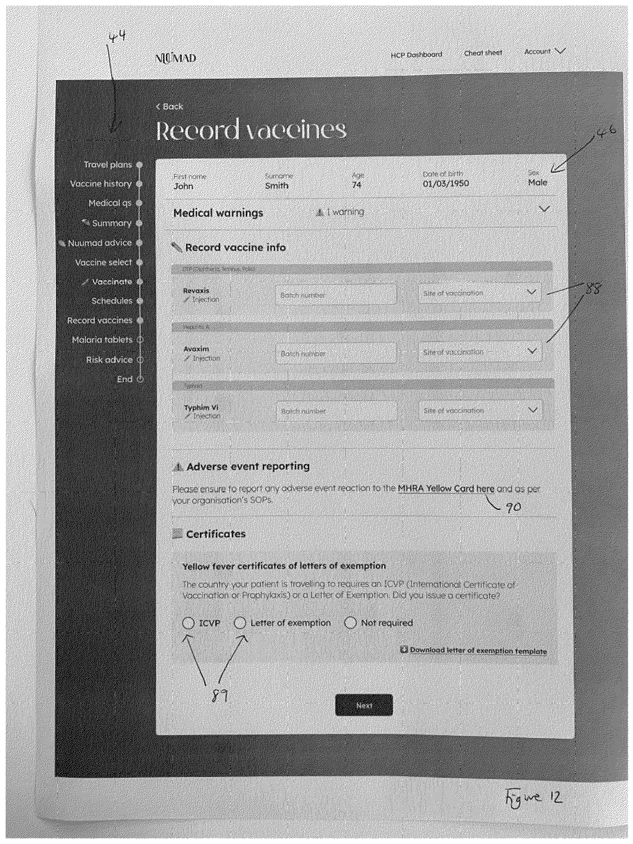

Figure 12 is an example of a screenshot on the graphical user interface 2 showing the vaccine record for the patient. A number of vaccine boxes 88 are shown with details of the vaccine given, the batch number, the site of vaccination and/or the like. A user completable report form or link 90 can be provided if required. A number of selectable options 89 are provided for the selection of either an International certificate, a letter of exemption and/or no certification required.

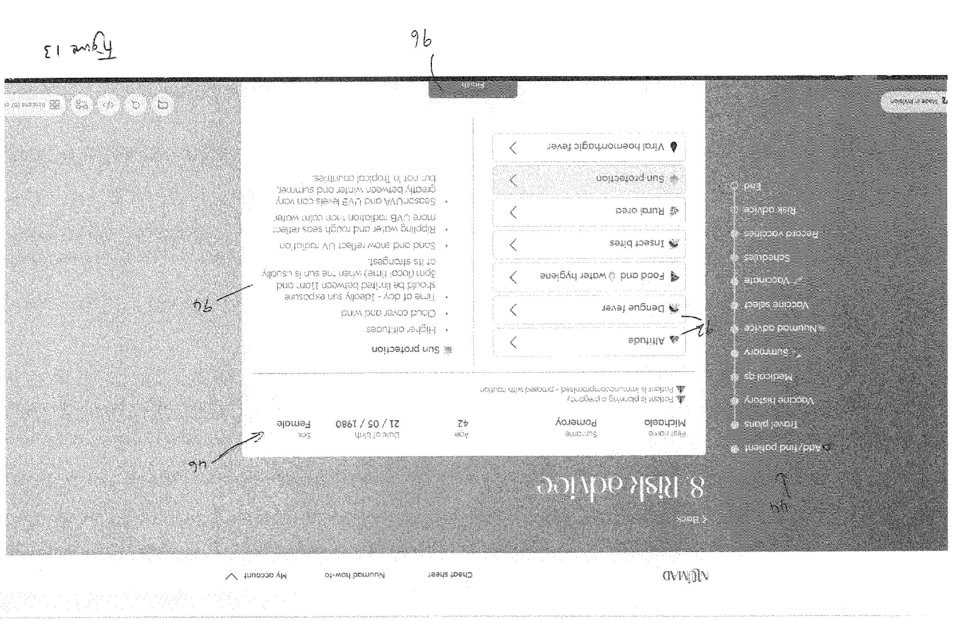

Figure 13 is an example of a screenshot on the graphical user interface showing non-vaccine preventable diseases, other travel risk advice and/or the like that can be generated and provided in the digital patient record and given to the patient. The additional travel risks are identified and set out in user selectable boxes 92. When a user selects one of the same, more detailed information on the selected travel risk is shown in section 94 of the screenshot. A user selectable finish button 96 is provided to exit the patient digital record.

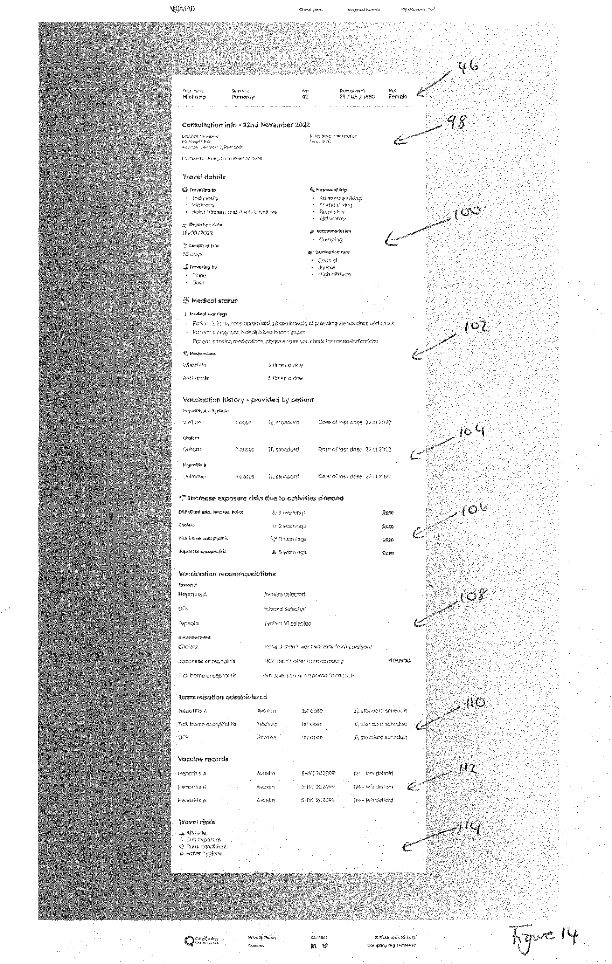

Figure 14 is an example of a screenshot on the graphical user interface showing a consultation data record for a patient. The patient data is shown in section 46.

Details of when and where the patient consultation took place and with which health care professional 98. Details of the travel destination, the length of stay, the purpose of the trip, the type of accommodation that will be used by the patient on the trip, the mode of transport, and the terrain at the travel destination are shown in section 100. Details of the patient's medical status is shown in section 102. Details of the patient's vaccination history is shown in section 104. Details of health risks related to the activities being carried out by the patient at the travel destination are shown in section 106. Details of vaccine recommendations are shown in section 108.

Details of vaccines administered are shown in section 110. Details of vaccine records are shown in section 112 and details of other non-vaccine preventable risks factors are shown in section 114.

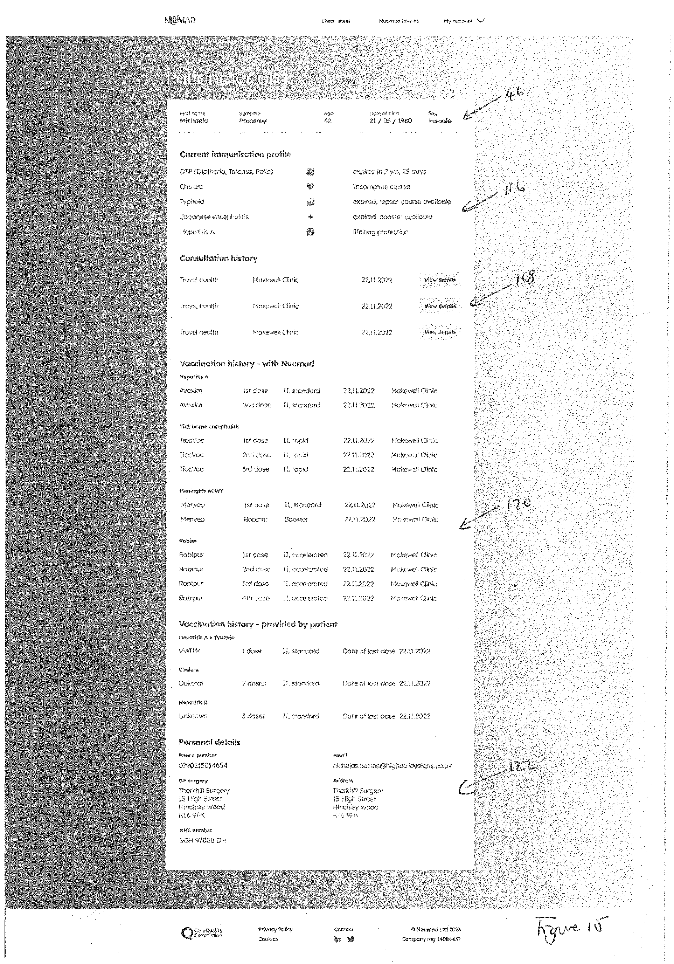

Figure 15 is an example of a screenshot on the graphical user interface showing a digital patient's data record. The record can include a section 116 on the patient's current immunisation profile, a section 118 of the patient's consultation history, a section 120 of the patient's vaccination history with the health care professional and elsewhere, and a section 122 of the patient's personal contact details and GP details.

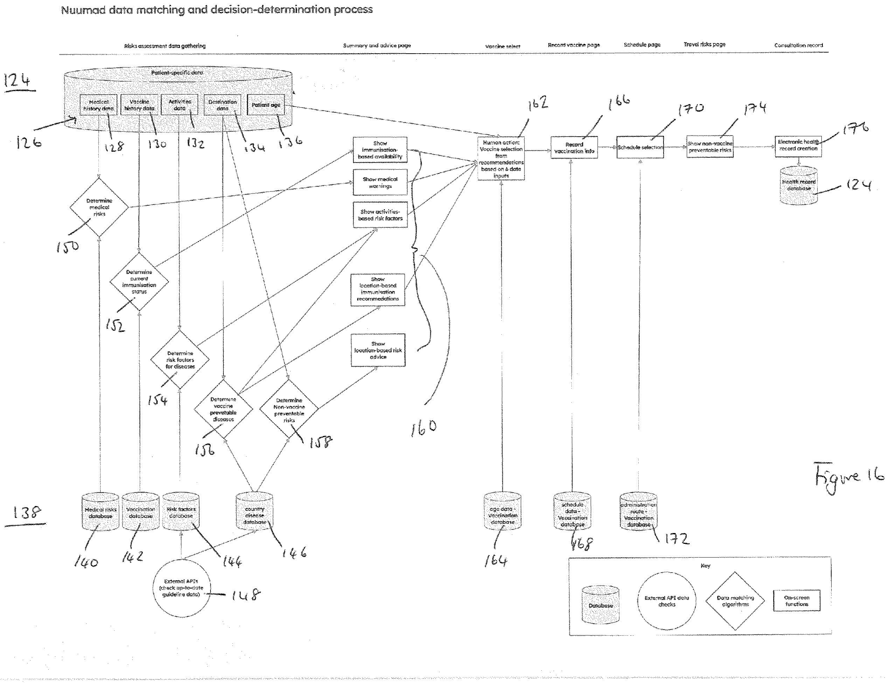

Figure 16 is a schematic showing the data matching process and decision-determination process that it undertaken by the digital platform according to one embodiment of the present invention. A first database 124 is shown containing a patient's digital data record 126, which in this example contains medical history data 128, vaccine history data 130, travel destination activity data 132, travel destination data 134, and patient's age data 136. At least a second database 138 is provided with country specific data, which in this example includes a medical risk database 140, a vaccination database 142, a risk factor database 144 and a country disease database 146. A further database or databases, such as for example external APIs, 148 communicate with the second set of databases 138 to update data in the same with the latest data. The digital platform according to the present invention is arranged to allow an algorithm based matching process to be undertaken identifying exact matching elements between the first and second database sets. The data matching algorithms being used in this example are for an exact match between the databases for medical risks 150, current immunisation status 152, risk factors for disease 154, vaccine preventable diseases 156 and non-vaccine preventable diseases and general travel risks 158.

Any exact matches identified by the algorithms are pulled out and summarised into a digital data record 160 or set of data records.

A further decision determination process can then take place on the digital data record 160 generated. This can take place automatically or manually via the digital platform. This further decision determination process involves making decisions on vaccine selection 162 using data from age related vaccination database 164, scheduling vaccination recommendations 166 using data from a schedule data vaccination database 168, scheduling a vaccination timetable 170 based on data from an administration route/ dosage requirements database 172, displaying non-vaccine preventable risks 174 and generating a digital patient health record 176, which can be stored in the first database of patient's digital records.

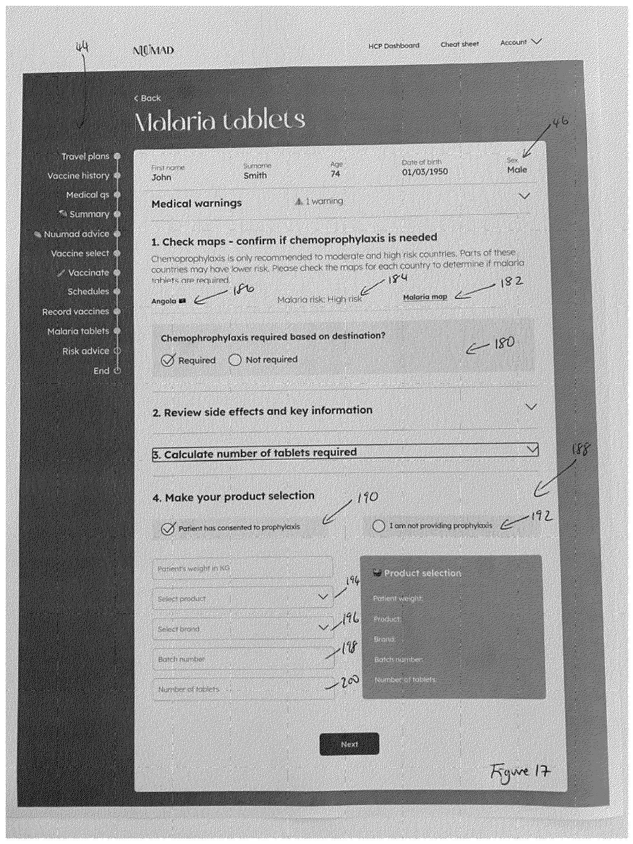

Figure 17 is an example of a screenshot on the graphical user interface showing a user selectable page for the option of providing malarial tablets. The page identifies whether chemoprophylaxis is required for malaria in the matched region of the world identified in the matching process using a tick box view 180. A malaria map 182 of the region can be selected for display, together with data on the risk of malaria 184 for the region 186.

A product selection section 188 is provided, showing a patient consent to treatment 190 (or not 192) and details of the product selected 194, brand selected 196, batch number of the medication 198 and the number of tablets 200.

Thus, it can be seen that the present invention allows the generation and storage of a digital patient data record, with a matching process available to automatically match a patient's intended travel destination(s) to health conditions and risk information, and particularly vaccination requirements, for that travel destination and to generate a vaccination treatment plan and schedule vaccination dates for selected vaccines.

It will be appreciated that any or any combination of the abovementioned features could be used to provide a digital platform with graphical user interface within the scope of the present invention.

## Claims

1. A digital platform, said digital platform including:
- at least one graphical user interface (GUI) for display on display means in use and for allowing input of data relating to an individual in use;
- the digital platform arranged to generate a digital data record including individual specific data based on the data input into the GUI in use;
- at least a first database associated with and/or communicatively coupled to the digital platform for storing the digital data record;
- at least a second database associated with and/or communicatively coupled to the digital platform for storing further digital data which comprises non-individual specific digital data; and
- processing means provided with and/or associated with the digital platform; wherein the processing means is arranged to undertake one or more data matching processes between data in the digital data record in the at least first database and the non-individual specific digital data in the at least second database, such that, on detection of one or more data matches based on one or more pre-determined data criteria between the digital data record and the non-individual specific digital data, a digital schedule and/or digital plan of action specific to the individual is automatically generated that is based on the one or more data matches of the one or more pre-determined data criteria in the at least first and second databases.

2. The digital platform according to claim 1, wherein the at least second databases comprises two or more databases, the one or more data matching processes undertaken by the processing means requires a data match to be identified between the individual's digital data record and each or at least two of the two or more databases in order to automatically generate the digital schedule and/or digital plan of action; and/or a data match is required between at least two of the pre-determined data criteria in the at least first and second databases in order to automatically generate the digital schedule and/or digital plan of action.

3. The digital platform according to any preceding claim, wherein one or more algorithms are provided with and/or associated with the digital platform and/or processing means to enable automatic generation of the digital schedule and/or the digital plan of action for the individual based on the data match(s).

4. The digital platform according to any preceding claim, wherein once at least one data match has been made between the digital data in the at least first and second databases, a sub-set of digital data is generated and/or identified and this sub-set of digital data is included in or forms the specific schedule and/or plan of action, forms a new digital data record specific to the individual and/or is added to or associated with the individual's digital data record.

5. The digital platform according to any preceding claim, wherein the data in the at least first and/or second databases and/or the matched data is ranked and/or ordered in order to generate the digital schedule and/or digital plan of action, or the most relevant data for matching or displaying.

6. The digital platform according to any preceding claim, wherein once the digital schedule and/or digital plan of action has been generated, the individual and/or a user of the digital platform can select, accept, adjust, modify and/or reject data or one or more parts of the schedule and/or plan of action.

7. The digital platform according to any preceding claim, wherein the generated digital schedule and/or digital plan of action includes or consists of any or any combination of a textual plan, one or more recommended products, one or more recommended vaccines, one or more recommended treatment plans, one or more dates, times, appointments, consultations, number of vaccine doses, types of vaccine immunisation available, or real time immunisation status.

8. The digital platform according to any preceding claim, wherein the digital schedule and/or digital plan of action is linked to a digital calendar or reminder system of the individual and/or user of the digital platform and/or the individual's digital data record.

9. The digital platform according to any preceding claim, including any or any combination of search means to allow a user and/or the individual to search data associated therewith or provided in the databases in use; navigational means to allow a user and/or the individual to navigate through different web pages or the databases in use; one or more selectable drop down menus, text boxes, selectable buttons, selectable links, options; a data summary or data dashboard; wherein the one or more data matching processes does not use probability, machine learning (ML), neural network links (NWL), data automation engines to analyse large data sets and/or artificial intelligence (AI) methods.

10. The digital platform according to any preceding claim, wherein the input data in the digital data record includes any or any combination of an individual's personal data; an individual's gender; an individual's contact details; an individual's health data; an individual's health status; and individual's medical background; an individual's medical history; one or more medications the individual is taking; one or more countries and/or geographical regions the individual intends to travel to within a pre-determined time frame, in the future or immediate future; one or more activities the individual intends to undertake within a pre-determined time period, in the future or immediate future; one or more accommodation types the individual intends to stay in within a pre-determined time frame, in the future or immediate future; one or more modes of transport the individual intends to use within a pre-determined time frame, in the future or immediate future; or vaccination history relating to the individual.

11. The digital platform according to any preceding claim, wherein the non-individual specific data relates to, consists or includes any or any combination of pre-verified data, one or more health conditions, diseases, vaccines, medical treatments, pharmaceutical products, therapeutic products, pre-programmed treatment of vaccine protocols, health risks; country and/or geographical region specific health conditions, disease, vaccine health risks; health conditions, diseases, medical treatments, pharmaceutical products, therapeutic products, vaccines or health risks associated with one or more activities; modes of transport; one or more types of accommodation, national and/or regional guidelines, national and/or regional statistics, data from the National Travel Health Network and Centre (NaTHNaC), Summary of Product Characteristics Data (SPC), national and/or regional guidelines, national vaccination guidelines, Green Book data including the latest information on vaccines and vaccination procedures for vaccine preventable infectious diseases in the UK, Centres for Disease Control and Prevention (CDC) data, World Health Organization (WHO) data, pre-programmed vaccine data, pre-programmed treatment plans, pre-programmes vaccine treatment plans, or Travax Data by Public Health Scotland.

12. The digital platform according to any preceding claim, wherein the one or more pre-determined data criteria includes any or any combination of a country and/or geographical region, a health condition, a disease, a medical treatment, a pharmaceutical product, a therapeutic product, a vaccine, a health risk, a mode of transport, a type of accommodation, an activity, gender, health status, an individual's age, or one or more medications.

13. The digital platform according to claim 3 wherein the one or more algorithms:
combines the matched non-individual specific data from the at least second databases to provide the digital schedule and/or digital plan of action; uses the matched data from the first and at least second databases to provide a risk assessment or identify one or more perceived risks to at least partly form the digital schedule and/or digital plan of action; calculate an individual's specific risks for travel diseases based on the individual's intended travel destination and/or medical history; calculate: immunisation status, patient vaccine availability or schedule availability; allow one or more vaccines to be ordered and/or offered; calculate vaccine availability for repeat consultations; forecast available vaccine schedules depending on an individual's prior vaccination history.

14. A digital system, said digital system comprising:
an electronic device;
a display screen provided with and/or associated with said electronic device;
a digital platform which is provided on, associated with and/or
communicatively coupled to the at least one electronic device;
said digital platform including:
- at least one graphical user interface (GUI) for display on the display screen in use and for allowing input of data relating to an individual in use;
- the digital platform arranged to generate a digital data record including individual specific data based on the data input into the GUI in use;
- at least a first database associated with and/or communicatively coupled to the digital platform for storing the digital data record;
- at least a second database associated with and/or communicatively coupled to the digital platform for storing further digital data which comprises non-individual specific digital data; and
- processing means provided with and/or associated with the digital platform;
wherein the processing means is arranged to undertake one or more data matching processes between data in the digital data record in the at least first database and the non-individual specific digital data in the at least second database, such that, on detection of one or more data matches based on one or more pre-determined data criteria between the digital data record and the non-individual specific digital data, a digital schedule and/or digital plan of action specific to the individual is automatically generated that is based on the one or more data matches of the one or more pre-determined data criteria in the at least first and second databases.

15. A method of using a digital platform, said digital platform including:
at least one graphical user interface (GUI) for display on display means in use and for allowing input of data relating to an individual in use; at least a first database associated with and/or communicatively coupled to the digital platform; at least a second database associated with and/or communicatively coupled to the digital platform for storing further digital data which comprises non-individual specific digital data; processing means provided with and/or
associated with the digital platform;
said method including the steps of:
- inputting data relating to an individual into the GUI;
- generating a digital data record including individual specific data based on the data input into the GUI;
- storing the digital data record in the at least first database;
wherein the processing means carries out the method steps of:
- undertaking one or more data matching processes between data in the digital data record in the at least first database and the non-individual specific digital data in the at least second database and, on detecting one or more data matches based on one or more pre-determined data criteria between the digital data record and the non-individual specific digital data, automatically generating a digital schedule and/or digital plan of action specific to the individual that is based on the one or more data matches of the one or more pre-determined data criteria in the at least first and second databases.
